# EUROPEAN PATENT SPECIFICATION

(11) **EP 3 937 867 B1**
(45) Date of publication and mention of the grant of the patent: **26.02.2025**
(21) Application number: 20769533.9
(22) Date of filing: 09.03.2020
(51) Int. Cl.: A61F 9/008

(54) **AUTOMATED LASER IRIDOTOMY**
AUTOMATISIERTE LASERIRIDOTOMIE
IRIDOTOMIE LASER AUTOMATISÉE

(30) Priority: 13.03.2019 US 201962817587 P
(43) Date of publication of application: 19.01.2022
(60) Divisional application: 25152129.0
(73) Proprietor: Belkin Vision Ltd., 8122214 Yavne (IL)
(72) Inventor: SACKS, Zachary S., 7173187 Modiin (IL); BELKIN, Michael, 5404402 Givat Shmu'el (IL)
(74) Representative: Beck Greener LLP
(86) International application number: PCT/IB2020/052020
(87) International publication number: WO 2020/183342

(56) References cited:
- WO-A1-01/95842
- WO-A1-2014/132162
- WO-A1-2018/232397
- WO-A1-93/16631
- WO-A1-93/16631
- RU-C1- 2 499 582
- RU-C1- 2 553 507
- US-A1- 2018 104 477
- US-B1- 6 325 792

## Description

### FIELD OF THE INVENTION

The present invention relates to ophthalmological procedures, particularly laser iridotomy.

### BACKGROUND

In a laser iridotomy procedure, a laser is used to form a hole in an iris. The hole facilitates increased fluid flow from behind the iris into the anterior chamber of the eye, thus relieving pressure on the optic nerve. Laser iridotomy may be used to treat or prevent conditions such as glaucoma, aqueous misdirection, and plateau iris syndrome.

International Patent Application Publication WO/2020/008323, describes a system including a radiation source and a controller. The controller is configured to display a live sequence of images of an eye of a patient. The controller is further configured to cause the radiation source, while displaying the sequence of images, to irradiate the eye with one or more aiming beams, which are visible in the images. The controller is further configured to receive a confirmation input from a user subsequently to causing the radiation source to irradiate the eye with the aiming beams, and in response to receiving the confirmation input, to treat the eye by causing the radiation source to irradiate respective target sites of the eye with a plurality of treatment beams.

US6325792 describes a system for irradiating the iris of an eye with laser radiation under control of controller.

### SUMMARY OF THE INVENTION

There is provided, in accordance with some embodiments of the present invention, a system including a laser, configured to irradiate a target site in an iris of an eye, a camera and a controller,

The controller is configured to identify, in one or more images of at least part of the iris, an indication of fluid flow through the target site, and in response to identifying the indication, inhibit the laser from further irradiating the target site.

In some embodiments, the target site is in a crypt in the iris.

In some embodiments, the controller is further configured to, prior to the laser irradiating the target site:
identify the crypt in another image of at least part of the iris, and
in response to identifying the crypt, designate at least a portion of the crypt as the target site.

In some embodiments, the controller is configured to identify the indication by identifying debris within a predefined distance from the target site.

In some embodiments, the controller is configured to identify the indication by identifying movement of the debris.

In some embodiments, the controller is configured to identify the indication by identifying that a size of the debris is greater than a predefined threshold.

In some embodiments, the controller is configured to identify the indication by identifying a hole through the target site.

In some embodiments, the controller is configured to identify the hole in response to a difference in color between the hole and a portion of the iris outside the hole.

In some embodiments, the controller is configured to identify the indication by identifying that a size of the hole is greater than a predefined threshold.

In some embodiments, the predefined threshold is a first predefined threshold, and the controller is configured to identify the indication by identifying debris within a predefined distance from the target site and having a size greater than a second predefined threshold.

In some embodiments, the second predefined threshold is a function of a size of the hole.

In some embodiments, the controller is further configured to:
identify the target site in other images of at least part of the iris, and
in response to identifying the target site, aim the laser at the target site.

There is further provided, in accordance with some embodiments of the present invention, a computer software product including a tangible non-transitory computer-readable medium in which program instructions are stored. The instructions, when read by a controller, cause the controller to identify, in one or more images of least part of an iris of an eye, an indication of fluid flow through a target site in the iris, and in response to identifying the indication, inhibit a laser from irradiating the target site.

The invention is defined by the appended claims.

The present invention will be more fully understood from the following detailed description of embodiments thereof, taken together with the drawings, in which:

### BRIEF DESCRIPTION OF THE DRAWINGS

Fig. 1 is a schematic illustration of a system for performing multiple procedures on an eye of a patient, in accordance with some embodiments of the present invention;
Fig. 2 is a schematic illustration of an ophthalmological surgical device, in accordance with some embodiments of the present invention;
Figs. 3A-B are schematic illustrations of aberration-correcting optics inserted into an optical path of a laser, in accordance with some embodiments of the present invention;
Fig. 4 is a flow diagram for a technique for performing an iridotomy procedure, in accordance with some embodiments of the present invention;
Fig. 5 is a schematic illustration of an eye and an image of a portion thereof, in accordance with some embodiments of the present invention;
Fig. 6 is a flow diagram for an algorithm for thinning a target site, in accordance with some embodiments of the present invention; and
Fig. 7 is a flow diagram for an algorithm for forming a hole through a target site, in accordance with some embodiments of the present invention.

### DETAILED DESCRIPTION OF EMBODIMENTS

### OVERVIEW

Conventional laser iridotomy procedures, which are performed manually, require substantial skill and training, and are often difficult to execute effectively and efficiently.

To address this challenge, embodiments of the present invention provide systems for automated laser iridotomy procedures. In particular, embodiments of the present invention provide a controller configured to control the firing of laser beams at a target site in an iris in response to processing images of the iris. For example, prior to firing the laser, the controller may identify a crypt in the iris through which a hole may be formed, and then designate a portion of the crypt as the target site. Subsequently, in response to tracking the target site in the images, the controller may keep the laser aimed at the target site while the beams are fired. Furthermore, in response to identifying, in the images, an indication of fluid flow through the target site, the controller may terminate the procedure.

Methods described throughout the description serve explanatory reasons only and do not form part of the invention.

Embodiments of the present invention further provide techniques for using the same laser both for thinning the targeted portion of the iris and for forming a hole through the targeted portion. For example, an actively Q-switched laser may be operated with relatively long pulses suitable for thinning the iris, and then with shorter pulses suitable for forming a hole in the iris. In contrast, conventional laser iridotomy procedures generally use separate lasers for thinning and hole formation.

Additionally, embodiments of the present invention provide techniques for using the same system to perform another type of automated procedure immediately before or after performing the automated iridotomy. For example, following an automated trabeculoplasty, the spot size of the laser beams may be reduced by moving the laser away from the patient and/or inserting a suitable optic into the optical path of the laser. Subsequently, the iridotomy may be performed with the reduced spot size.

### SYSTEM DESCRIPTION

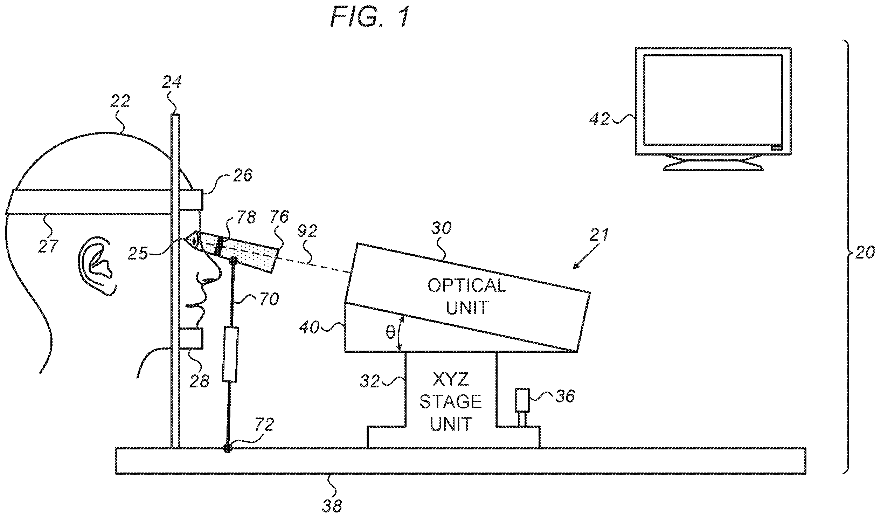

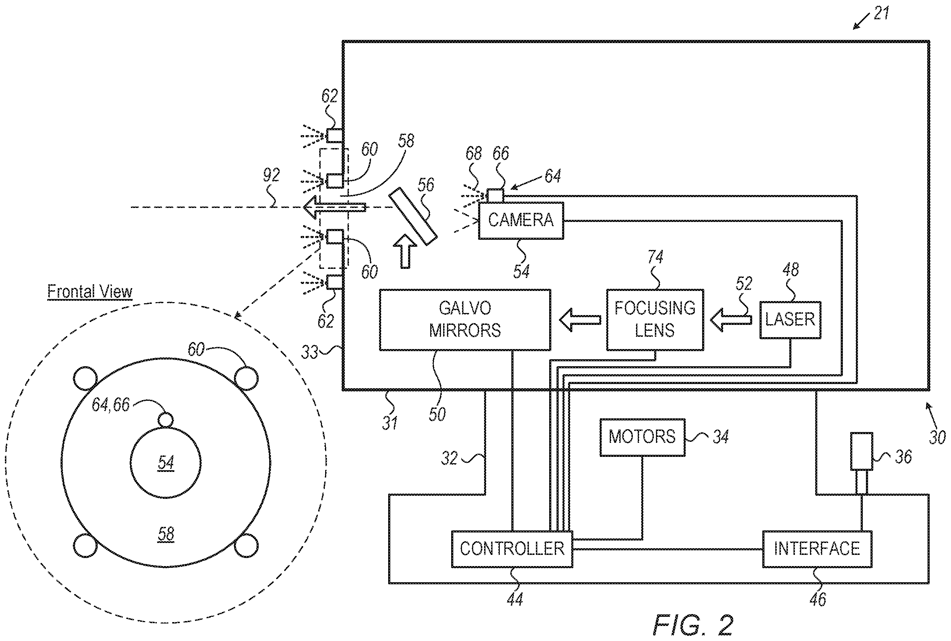

Reference is initially made to Fig. 1, which is a schematic illustration of a system 20, comprising an ophthalmological surgical device 21, for performing multiple surgical procedures on an eye 25 of a patient 22, in accordance with some embodiments of the present invention. Reference is further made to Fig. 2, which is a schematic illustration of ophthalmological surgical device 21, in accordance with some embodiments of the present invention.

Ophthalmological surgical device 21 comprises an optical unit 30 and a controller 44. Optical unit 30 comprises one or more beam-directing elements, comprising, for example, one or more galvo mirrors 50, which may be referred to collectively as a "galvo scanner," or a mirror moved by acoustic coils. Alternatively or additionally, the beam-directing elements may comprise a beam combiner 56. Optical unit 30 further comprises a laser 48, which is configured to irradiate eye 25 with treatment beams 52 by emitting treatment beams 52 toward the beam-directing elements such that the beams are directed by the beam-directing elements toward the eye. Each treatment beam 52 may have an elliptical (e.g., a circular) shape, a square shape, or any other suitable shape. In some embodiments, each treatment beam includes a pattern of beamlets, such as a spiral or circle of circular beamlets.

More specifically, before the emission of each treatment beam 52 (or each series of treatment beams) from laser 48, and/or while the beam is being emitted, controller 44 aims the beam-directing elements at a target site on eye 25 such that the beam is directed, by the beam-directing elements, toward the target site. For example, the beam may be deflected by galvo mirrors 50 toward beam combiner 56, and then deflected by the beam combiner such that the beam impinges on the target site. (Since each treatment beam impinges on the eye with a non-infinitesimal spot size, the present application generally describes each beam as impinging on a "site" or "region" of the eye, whose area is a function of the spot size, rather than impinging at a "point" on the eye.) The beam thus follows an optical path 92, which extends from the most downstream of the beam-directing elements - such as beam combiner 56 - to eye 25. (In practice, the respective paths of successive beams may vary slightly from each other, e.g., due to the beams being aimed at different respective target sites and/or due to movement of the eye; however, given that this variation is very slight, the present description refers to a single optical path 92 followed by all the treatment beams.)

Typically, optical unit 30 further comprises a focusing lens 74, which focuses treatment beams 52. In some embodiments, as shown in Fig. 2, focusing lens 74 is situated upstream from beam-directing elements, e.g., between the laser and the beam-directing elements. In other embodiments, the focusing lens is situated downstream from one or more of the beam-directing elements, e.g., between galvo mirrors 50 and beam combiner 56.

Optical unit 30 further comprises a camera 54. As shown in Fig. 2, camera 54 is typically aligned, at least approximately, with optical path 92; for example, the angle between optical path 92 and a hypothetical line extending from eye 25 to the camera may be less than 15 degrees. In some embodiments, the camera is positioned behind beam combiner 56, such that the camera receives light via the beam combiner. In other embodiments, the camera is not aligned with optical path 92, such that light reaching the camera does not pass through the beam combiner.

Before each procedure, camera 54 acquires at least one image of eye 25. Based on the image, controller 44 and/or a user of the system, such as an ophthalmologist or another physician, may define and/or modify one or more target sites that are to be irradiated. Subsequently, during the procedure, camera 54 may acquire multiple images of the patient's eye at a relatively high frequency. By processing each of these images, controller 44 may track any movement of the eye and/or assess the effects of the treatment thus far. In response thereto, the controller may control laser 48 and the beam-directing elements.

Typically, laser 48 is further configured to emit one or more aiming beams at the eye, e.g., as described in International Patent Application Publication WO/2020/008323. The aiming beams, which are visible in the images acquired by camera 54, may help controller 44 control laser 48 and the beam-directing elements. Typically, the aiming beams are collinear with treatment beams 52.

Typically, optical unit 30 further comprises a light source 66, which is aligned, at least approximately, with optical path 92. Light source 66 is configured to function as a fixation target 64 by transmitting visible light 68, as described in International Patent Application PCT/IB2019/059058. In other embodiments, the optical unit does not comprise light source 66.

Typically, the optical unit comprises an optical bench, and at least some of the aforementioned optical components belonging to the optical unit, such as the laser, the galvo mirrors, and the beam combiner, are coupled to the optical bench. Typically, the optical unit further comprises a front face 33, through which the treatment beams, aiming beams, and visible light 68 pass. For example, optical unit 30 may comprise an encasement 31, which at least partially encases the optical bench and comprises front face 33. (Encasement 31 may be made of a plastic, a metal, and/or any other suitable material.) Alternatively, front face 33 may be attached to, or may be an integral part of, the optical bench.

In some embodiments, front face 33 is shaped to define an opening 58, through which the treatment beams, aiming beams, and visible light 68 pass. In other embodiments, the front face comprises an exit window in lieu of opening 58, such that the aforementioned radiation passes through the exit window. The exit window may be made of a plastic, a glass, or any other suitable material.

Typically, optical unit 30 further comprises one or more illumination sources 60 comprising, for example, one or more LEDs, such as white-light or infrared LEDs. In such embodiments, controller 44 may cause illumination sources 60 to intermittently flash light at the eye, as described in International Patent Application Publication WO/2020/008323, whose disclosure is incorporated herein by reference. This flashing may facilitate the imaging performed by the camera, and may further help constrict the pupil of the eye. (For ease of illustration, the electrical connection between controller 44 and illumination sources 60 is not shown explicitly in Fig. 2.) In some embodiments, illumination sources 60 are coupled to front face 33, as shown in Fig. 2.

To facilitate positioning the optical unit, the optical unit may comprise a plurality of beam emitters 62 (comprising, for example, respective laser diodes), which are configured to shine a plurality of triangulating range-finding beams on the eye, e.g., as described in International Patent Application Publication WO/2020/008323. In some embodiments, beam emitters 62 are coupled to front face 33, as shown in Fig. 2. In other embodiments, beam emitters 62 are coupled directly to the optical bench. (In such embodiments, the range-finding beams may be emitted through opening 58.)

Optical unit 30 is mounted onto an XYZ stage unit 32, which is controlled by a control mechanism 36, such as a joystick. Using control mechanism 36, the user of system 20 may position the optical unit along one or more of the optical unit's three axes of movement prior to treating the eye. In some embodiments, XYZ stage unit 32 comprises locking elements configured to inhibit motion of the stage unit following the positioning of the stage unit.

In some embodiments, XYZ stage unit 32 comprises one or more motors 34, and control mechanism 36 is connected to interface circuitry 46. As the user manipulates the control mechanism, interface circuitry 46 translates this activity into appropriate electronic signals, and outputs these signals to controller 44. In response to the signals, the controller controls the motors of the XYZ stage unit.

In other embodiments, XYZ stage unit 32 is controlled manually by manipulating the control mechanism. In such embodiments, the XYZ stage unit may comprise a set of gears instead of motors 34.

System 20 further comprises a headrest 24, typically comprising a forehead rest 26 and a chinrest 28. During each procedure, patient 22 rests his head against headrest 24 such that the headrest supports the head of the patient; for example, the patient may press his forehead against forehead rest 26 while resting his chin on chinrest 28. In some embodiments, headrest 24 comprises an immobilization strap 27, configured to secure the patient's head from behind and thus keep the patient's head pressed against the headrest. Typically, headrest 24 is coupled to a surface 38, such as a tray or tabletop. In some embodiments, XYZ stage unit 32 is also coupled to surface 38.

In some embodiments, as shown in Fig. 1, while irradiating the patient's eye, the optical unit is directed obliquely upward toward the eye while the eye gazes obliquely downward toward the optical unit, such that optical path 92 is oblique. For example, the optical path may be oriented at an angle θ of between five and twenty degrees with respect to the horizontal. Advantageously, this orientation reduces occlusion of the patient's eye by the patient's upper eyelid and associated anatomy.

In some embodiments, as shown in Fig. 1, the oblique orientation of the optical path is achieved by virtue of the optical unit being mounted on a wedge 40, which is mounted on the XYZ stage unit. In other words, the optical unit is mounted onto the XYZ stage unit via wedge 40. (Wedge 40 is omitted from Fig. 2.)

In some embodiments, system 20 further comprises a radiation conduit 76, and the various types of radiation described herein, such as treatment beams 52 and visible light 68, are emitted through conduit 76 (i.e., are emitted such that the beams of radiation pass through the conduit). In some embodiments, the inner and/or outer surface of conduit 76 is configured to absorb any misaimed treatment beams or scattered light, and/or to block any external light from interfering with the camera. Alternatively or additionally, the conduit may hold one or more optics, as further described below.

Typically, conduit 76 is hollow, such that the radiation passes through air within the conduit. For example, conduit 76 may comprise a frustum-shaped or cylindrically-shaped tube, which may be made from metal, glass, or any other suitable material. In some such embodiments, the conduit comprises a continuous wall. In other such embodiments, conduit 76 is shaped to define one or more openings. For example, conduit 76 may comprise a wire structure, such as a wire mesh. In other embodiments, conduit 76 comprises a frustum-shaped or cylindrically-shaped transparent piece of material (e.g., glass), such that the radiation passes through the material.

In some embodiments, the distal end of conduit 76 contacts the eye. In such embodiments, the distal end of the conduit may retract the eyelids of eye 25 and/or stabilize the eye, i.e., inhibit the eye from moving. In other embodiments, the conduit does not contact the eye.

Typically, conduit 76 is not coupled to device 21, such that the distance between laser 48 and the eye may be adjusted without moving the conduit. For example, the conduit may be coupled to headrest 24 or to surface 38. As a specific example, the conduit may be coupled to surface 38 via a telescoping arm 70 comprising one or more pivot joints 72. By adjusting the length of arm 70 and/or rotating pivot joints 72, the user may adjust the location and orientation of the conduit. Alternatively, conduit 76 may be held by the user.

System 20 further comprises a monitor 42, configured to display images of the eye acquired by the camera. Monitor 42 may be attached to optical unit 30 or disposed at any other suitable location, such as on surface 38 next to device 21. In some embodiments, monitor 42 comprises a touch screen, and the user inputs commands to the system via the touch screen. Alternatively or additionally, system 20 may comprise any other suitable input devices, such as a keyboard or a mouse, which may be used by the user.

In some embodiments, monitor 42 is connected directly to controller 44 over a wired or wireless communication interface. In other embodiments, monitor 42 is connected to controller 44 via an external processor, such as a processor belonging to a standard desktop computer.

In some embodiments, as shown in Fig. 2, controller 44 is disposed within XYZ stage unit 32. In other embodiments, controller 44 is disposed externally to the XYZ stage unit. Alternatively or additionally, the controller may cooperatively perform at least some of the functionality described herein with another, external processor.

In some embodiments, at least some of the functionality of controller 44, as described herein, is implemented in hardware, e.g., using one or more Application-Specific Integrated Circuits (ASICs) or Field-Programmable Gate Arrays (FPGAs). Alternatively or additionally, controller 44 may perform at least some of the functionality described herein by executing software and/or firmware code. For example, controller 44 may comprise a central processing unit (CPU) and random access memory (RAM). Program code, including software programs, and/or data may be loaded into the RAM for execution and processing by the CPU. The program code and/or data may be downloaded to the controller in electronic form, over a network, for example. Alternatively or additionally, the program code and/or data may be provided and/or stored on non-transitory tangible media, such as magnetic, optical, or electronic memory. Such program code and/or data, when provided to the controller, produce a machine or special-purpose computer, configured to perform the tasks described herein. In some embodiments, the controller comprises a system on module (SOM), such as the Varisite^{™} DART-MX8M.

### PERFORMING MULTIPLE PROCEDURES

Advantageously, system 20 is configured to perform multiple procedures on eye 25 in a single session, with relatively little delay between the procedures. For example, a second procedure may be started, i.e., the first treatment beam of the second procedure may be emitted, within five minutes of stopping to perform a first procedure, i.e., within five minutes of emitting the last treatment beam of the first procedure.

For example, an iridotomy may be performed immediately following another procedure, such as a trabeculoplasty. (The trabeculoplasty may be performed automatically or semiautomatically, e.g., as described in International Patent Application Publication WO/2020/008323 and International Patent Application PCT/IB2019/059058). Alternatively, the iridotomy may be performed immediately prior to the other procedure.

To facilitate performing the successive procedures, system 20 allows changing the spot size, on the eye, of the treatment beams emitted by the laser. Thus, for example, following a trabeculoplasty, the spot size may be decreased (e.g., by at least 70 microns, such as at least 300 microns) to a value suitable for iridotomy procedures. (Typically, the required spot size for an iridotomy is relatively small, due to the high intensities required for photodisruption.) Subsequently, an iridotomy may be performed with the decreased spot size. Alternatively, following an iridotomy, the spot size may be increased, and a trabeculoplasty may then be performed with the increased spot size.

As a specific example, during a trabeculoplasty, the spot size on the eye may be greater than 100 microns, such as greater than 300 microns (e.g., approximately 400 microns). Subsequently, the spot size may be decreased to less than 30 microns, such as less than 10 microns, and an iridotomy may then be performed.

Various techniques may be used to change the spot size. For example, controller 44 may move focusing lens 74 toward or away from the laser. Alternatively or additionally, the user may adjust the distance between the laser and the eye by moving the optical unit toward or away from the patient. (To facilitate this adjustment, the controller may adjust the angle of convergence between the range-finding beams emitted by beam emitters 62 such that the range-finding beams converge on the eye when the optical unit is at the correct distance from the patient.) To compensate for the movement of the optical unit, controller 44 may adjust the focus of the camera, e.g., by moving another focusing lens (not shown) toward or away from the imaging sensor of the camera such that the portion of the eye that is to be irradiated is in focus.

Alternatively or additionally, to change the spot size, an optic 78, which modifies the convergence angle of the treatment beams, may be placed in optical path 92. Optic 78 may comprise any number of lenses (e.g., singlet, doublet, and/or triplet lenses), diffractive optical elements, freeform optical elements, phase plates, and/or other optical elements.

Typically, following the placement of optic 78 in optical path 92, the optic also lies in the optical path of the camera such that, while the eye is irradiated during the second procedure, the eye is imaged through the optic. Thus, advantageously, the optic may magnify or demagnify the target site in the field of view of the camera. For example, for an iridotomy, optic 78 may reduce the spot size of the laser and also cause the camera to zoom in to the target site.

In some embodiments, optic 78 is contained in conduit 76. For example, following the performance of the first procedure without the conduit, the conduit, while containing the optic, may be placed between the laser and the eye, and the second procedure may then be performed. Alternatively, following the performance of the first procedure with the conduit but without the optic, the optic may be placed in the conduit, and the second procedure may then be performed. Optic 78 may be coupled to the inner wall of conduit 76 via an adhesive, via one or more screws, and/or via any other suitable coupling mechanism.

In other embodiments, optic 78 is attached to the exit window or to the optical bench of the optical unit, or is otherwise placed (e.g., automatically, by the controller) in the optical path of the laser.

Typically, the wavelength of treatment beams 52 is between 300 and 1300 nm, such as between 500 and 1100 nm. In some embodiments, the wavelength used for an iridotomy is different from the wavelength used for another procedure performed before or after the iridotomy. As a purely illustrative example, following a trabeculoplasty at 532 nm, an iridotomy may be performed at 1064 nm. In other embodiments, the wavelength is the same; for example, following a trabeculoplasty at 532 nm, an iridotomy may also be performed at 532 nm.

(It is noted that 1064 nm is conventionally used for iridotomy procedures. However, the present inventors realized that radiation beams having a smaller wavelength, such as 532 nm, may better facilitate the photodisruption process required for iridotomy, by virtue of such beams possessing a smaller spot size (and hence, greater intensity) and greater photon energy relative to larger-wavelength beams.)

In some embodiments, prior to performing an iridotomy, an aberration-correcting optic is inserted into the optical path of the laser. The aberration-correcting optic corrects for any off-axis aberrations of the laser beams caused by the laser beams passing through the cornea of the eye at a displacement from the center of the cornea. Typically, following the insertion of the aberration-correcting optic into the optical path of the laser, the aberration-correcting optic lies also in the optical path of the camera.

For example, optic 78, which modifies the spot size of the laser beams as described above, may also function as an aberration-correcting optic. For embodiments in which optic 78 is not used or in which optic 78 provides insufficient aberration correction, another aberration-correcting optic may be used. Such an optic may comprise a lens that is pressed against the eye, such as an Abraham lens or a Wise lens. Such a lens may be coupled to the distal end of conduit 76 or held by the user of the system. Since such a lens typically affects the spot size of the radiation beams emitted by the laser, the adjustment of the spot size is performed differently for embodiments in which the lens is used, relative to other embodiments in which the lens is not used.

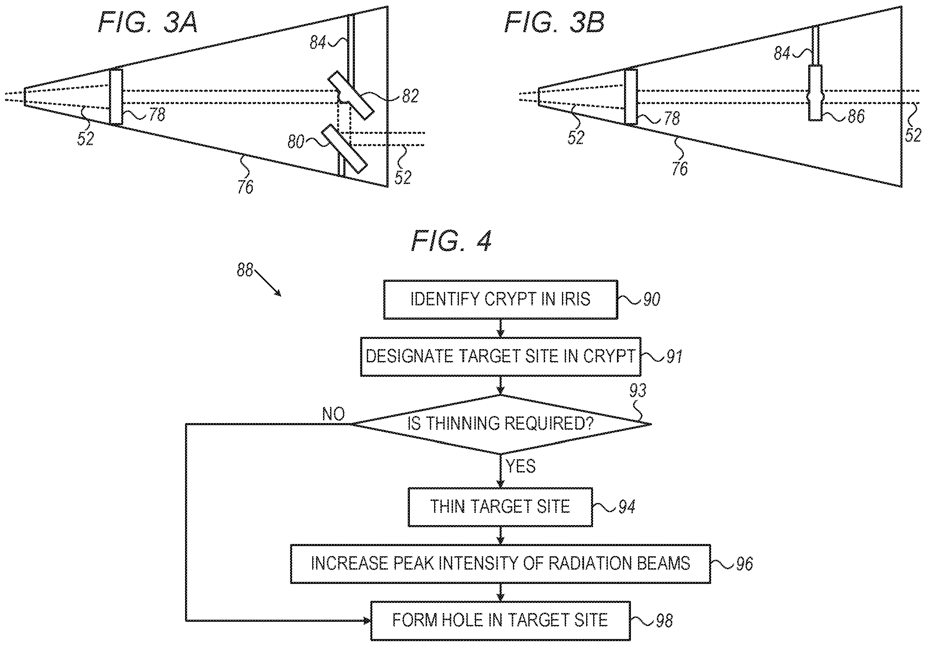

Alternatively to a contact lens, the aberration-correcting optic may comprise an optic that does not contact the eye. Such an optic may be inserted into the optical path of the laser in any suitable way, as described above for optic 78. For examples of such embodiments, reference is now made to Figs. 3A-B, which are schematic illustrations of aberration-correcting optics inserted into the optical path of the laser, in accordance with some embodiments of the present invention.

In some embodiments, a deformable or elliptical mirror 82 is inserted into the optical path. For example, as shown in Fig. 3A, mirror 82 may be coupled to the inner wall of conduit 76 by a rod 84. Conduit 76 may further contain another mirror 80, which deflects treatment beams 52 onto mirror 82.

In other embodiments, a freeform optic is inserted into the optical path. Such an optic may comprise a transmissive phase element 86 (comprising a lens, for example), as shown in Fig. 3B.

Optic 78 may be situated upstream or downstream from the aberration-correcting optic. As described above with reference to Fig. 1 and illustrated in Figs. 3A-B, optic 78 may reduce the spot size of the treatment beams to a value suitable for iridotomy.

Advantageously, the optics inserted into the optical path of the laser are also configured to facilitate the use of visible light 68 (Fig. 2), the range-finding beams, the aiming beams, and the light emitted by illumination sources 60 (Fig. 2). For example, optic 78 may change the spot size of the aiming beams by the same factor by which the spot size of the treatment beams is modified. Conversely, the range-finding beams and visible light 68 may pass through optic 78 without any modification.

In alternative embodiments, the aberration-correcting optic is inserted into the optical path of the camera but not that of the laser.

Alternatively or additionally to any of the elements described above, the aberration-correcting optic may comprise any number of lenses (e.g., singlet, doublet, and/or triplet lenses), diffractive optical elements, freeform optical elements, phase plates, and/or other optical elements.

### IRIDOTOMY PROCEDURES

Reference is now made to Fig. 4, which is a flow diagram for a technique 88 for performing an iridotomy procedure, in accordance with some embodiments of the present invention.

It is typically easiest and safest to form a hole through a crypt, rather than through other portions of the iris. Hence, technique 88 typically begins with a crypt-identifying step 90, at which a crypt in the iris is identified for irradiation.

In some embodiments, crypt-identifying step 90 is performed automatically by controller 44 (Fig. 2), in that the controller identifies the crypt in an image, acquired by the camera, of at least part of the iris. In general, any suitable image-processing techniques may be used for this identification. For example, the controller may first adjust the brightness, contrast, uniformity, and/or white balance of the image. Subsequently, the controller may deblur and remove any noise from the image. Next, the controller may apply an edge detector to the image, and then use the detected edges to segment any objects within the iris. Subsequently, open objects, objects whose distance to the pupil of the eye is less than a predefined threshold (e.g., one third of the distance between pupil and the limbus), and/or objects whose size is less than a predefined threshold (e.g., whose maximum width is less than 30 µm) may be discarded. (To facilitate removing small and open objects, the controller may use filling and erosion techniques.) Subsequently, the controller may select one of the remaining objects, which are assumed to be crypts. For example, the controller may select the largest crypt within ±30 degrees of the top of the iris, i.e., between 11 o'clock and 1 o'clock, the largest crypt within a predefined number of degrees from 3 o'clock or 9 o'clock, or the largest crypt in the upper half of the iris.

Typically, following the identification of the crypt, the controller asks the user to confirm the selection of the crypt for irradiation. For example, the controller may display the image on monitor 42 (Fig. 1) with the identified crypt highlighted, and the user may then use a suitable user interface to confirm the identified crypt or to select a different crypt.

In other embodiments, the user identifies a crypt in the image, and then uses a suitable user interface to indicate the location of the crypt to the controller.

In response to the identification of the crypt, the controller, at a target-site-designating step 91, designates a portion of the crypt (or the entire crypt) as the target site. Optionally, the controller may then indicate the target site to the user, and the user may then confirm or move the target site.

Next, at a thickness-assessing step 93, the controller ascertains whether thinning of the target site is required. For example, the controller may receive an input from the user specifying whether thinning is required. Alternatively, the controller may perform this assessment automatically, based on the color of the iris. (Typically, brown irises are thicker than irises of other colors; hence, brown irises may require thinning whereas other irises may not.) Optionally, the controller may then ask the user to confirm the controller's assessment.

In response to ascertaining that thinning is required, the controller, at a thinning step 94, causes the laser to thin the target site by irradiating the target site with one or more first radiation-pulses such that the first radiation-pulses cauterize the target site. In some embodiments, as described below, the spot size for thinning is larger than the spot size for the subsequent hole formation, such that the thinned portion of the iris extends beyond the target site. Hence, prior to performing thinning step 94, the controller may indicate, to the user, the portion of the iris that is to be thinned, and ask the user for confirmation.

Subsequently to thinning the target site, the controller, at a peak-intensity-increasing step 96, increases the peak intensity of the radiation beams emitted by the laser, such that the peak intensity is sufficiently high to cause photodisruption of the iris. Following peak-intensity-increasing step 96, or if no thinning is required, the controller, at a hole-forming step 98, causes the laser to form a hole through the target site by irradiating the iris with one or more second radiation-pulses.

In some embodiments, the peak intensity is increased by decreasing the pulse duration of the laser such that each pulse delivers the same amount of energy over less time. For example, while the duration of each of the first radiation-pulses may be between 100 ns and 100 ms, the duration of each of the second radiation-pulses may be less than 10 ns.

In some embodiments, the laser comprises an actively Q-switched laser including a Q-switch. Conventionally, such lasers have been used only for relatively short pulses, and other types of lasers have been used for iris thinning. However, the present inventors realized that an actively Q-switched laser may be used for thinning, by leaving the Q-switch open for a relatively long time, e.g., at least 100 ns, for each of the first radiation-pulses, optionally while continuing to pump (i.e., deliver energy to) the laser while the Q-switch is open.

In other embodiments, the laser comprises a fiber laser. To thin the targeted portion of the iris, the controller may operate the laser in a longer-pulse mode. Subsequently, the controller may change the operating setting of the laser to a shorter-pulse mode, and then use the laser to form a hole in the targeted portion.

Alternatively or additionally to shortening the pulse duration, the controller may increase the peak intensity by reducing the spot size of the radiation beams on the iris. This may be done, for example, by moving focusing lens 74 (Fig. 2).

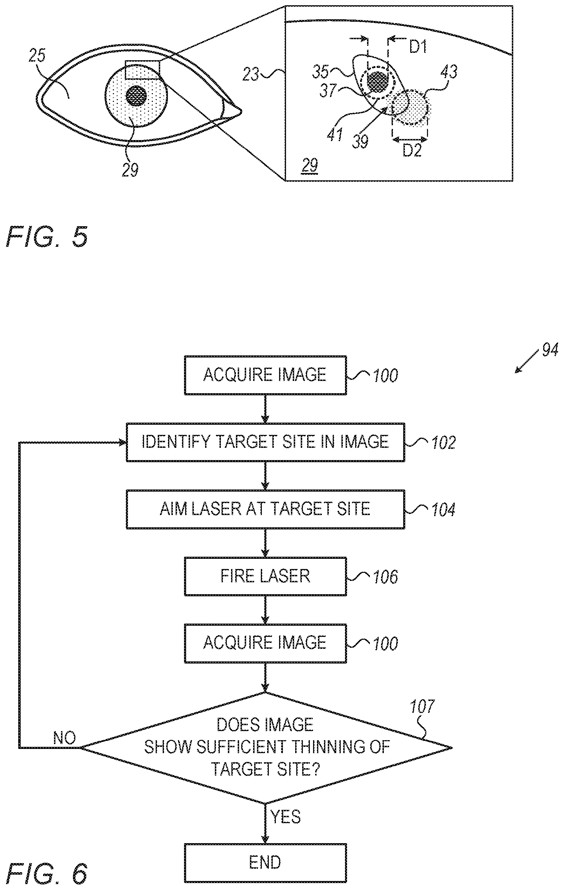

Reference is now made to Fig. 5, which a schematic illustration of eye 25 and an image 23 of a portion thereof, in accordance with some embodiments of the present invention.

Typically, at hole-forming step 98 (Fig. 4), images 23 showing the target site 41 are acquired at a relatively high frequency while target site 41 is irradiated. The controller processes the images so as to check for an indication of fluid flow through the target site. In response to identifying an indication of fluid flow, the controller terminates the procedure, i.e., inhibits the laser from further irradiating the target site.

For example, the controller may check whether an image shows a hole 37 through the target site. Hole 37 may be identified, for example, in response to a difference in color between the hole and a portion of the iris 29 outside the hole, such as an adjacent portion of the crypt 35 in which the target site is located, and/or in response to comparing the image with a reference image acquired prior to the procedure. To stop the procedure, the controller may require that the size of any identified hole be greater than a predefined threshold. For example, the controller may require that the diameter D1 of the hole be greater than a threshold diameter that is between 150 and 750 µm, such as between 150-200 µm or between 350-600 µm.

Alternatively or additionally, the controller may check each acquired image for any debris 39 within a predefined distance from the target site, given that the presence of debris 39 near the target site may be indicative of fluid flow through the target site. (Typically, debris 39 appears in the image as a cloud of small particles.) To facilitate identifying debris 39, the controller may compare each acquired image with a reference image acquired prior to the procedure, using any suitable image-registration techniques to align the two images with one another. To stop the procedure, the controller may require that the size of any identified debris be greater than a predefined threshold. For example, the controller may compute the area covered by the debris and require that this area be greater than a threshold area. Alternatively, the controller may fit a circle 43 to the identified debris, and require that the diameter D2 of circle 43 be at least a predefined multiple of diameter D1. This multiple may be between two and eight, or greater than eight, for example.

In some embodiments, the controller further checks for movement of the debris, by comparing the image with a previously-acquired image, e.g., using optical-flow techniques. (Specifically, the controller may check for movement of debris away from the target site, given that fluid is expected to flow from behind the iris.) In such embodiments, to stop the procedure, the controller may require movement of the debris, alternatively or additionally to requiring that the size of the debris be greater than a threshold.

Alternatively or additionally to checking for fluid flow, the controller may use the acquired images to check for any movement of the eye and/or for any changes in morphology resulting from the treatment thus far. In response to identifying movement of the eye and/or changes in morphology, the controller may adjust the beam-directing elements such that the laser remains aimed at the target site.

### EXAMPLE ALGORITHMS

Reference is now made to Fig. 6, which is a flow diagram for an algorithm for thinning the target site at thinning step 94, in accordance with some embodiments of the present invention.

Per Fig. 6, thinning step 94 begins with an image-acquiring step 100, at which camera 54 (Fig. 2) acquires an image of at least part of the iris, the image typically being centered approximately at the last identified location of the target site. Following image-acquiring step 100, the controller, at a target-site-identifying step 102, identifies the target site in the acquired image. For example, the controller may use pattern-matching techniques to identify the crypt in which the target site is located and/or to identify other features of the iris overlapping with, or situated near, the target site.

As described above with reference to Figs. 1-2, optic 78 may magnify the iris in the field of view of the camera, thus facilitating identifying the target site.

Following the identification of the target site, the controller, at a laser-aiming step 104, aims the laser at the target site. The controller then fires the laser at a laser-firing step 106, such that treatment beams are emitted by the laser. The treatment beams may include, for example, a sequence of three or more pulses having a repetition rate of at least 20 Hz.

Although not shown in Fig. 6, the controller typically uses an aiming beam to verify that the laser is aimed at the target site, as described above with reference to Figs. 1-2. For example, subsequently to aiming the laser, the controller may cause the laser to fire an aiming beam at the eye. Subsequently, the controller may acquire another image, and then verify that the aiming beam coincides with the target site in the image. Alternatively or additionally, the controller may ascertain the spot size of the aiming beam in the image. In response to the spot size deviating from the desired spot size, the controller may adjust the focus of the laser by moving focusing lens 74 (Fig. 2), and/or output a message to the user indicating that the optical unit should be moved toward or away from the patient. Alternatively or additionally, based on the image of the aiming beam, the controller may adjust the focus of the camera.

Following the firing of the laser, image-acquiring step 100 is again performed. Subsequently, the controller, at a thinning-assessment step 107, ascertains whether the acquired image shows sufficient thinning of the target site. For example, by comparing the image to previously-acquired images, the controller may check for changes in the properties of the iris at or near the target site. For example, the controller may check for changes in color, new or missing edges, and/or changes in the size or shape of the crypt. In response to ascertaining that the targeted portion of the iris is sufficiently thinned, the controller terminates the thinning procedure. Otherwise, the controller returns to target-site-identifying step 102.

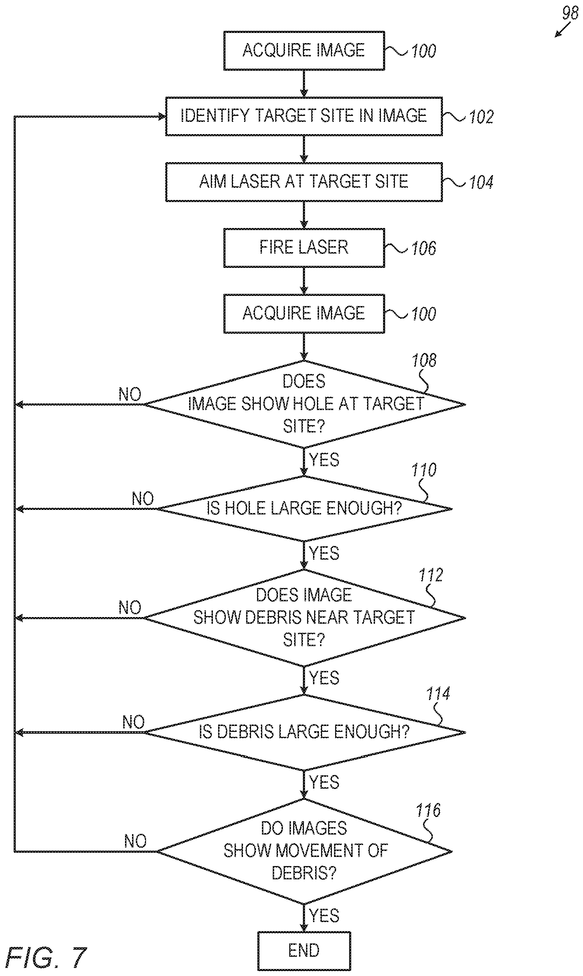

Reference is now made to Fig. 7, which is a flow diagram for an algorithm for forming a hole through the target site at hole-forming step 98, in accordance with some embodiments of the present invention.

Per Fig. 7, hole-forming step 98 begins with image-acquiring step 100. Following image-acquiring step 100, the controller, at target-site-identifying step 102, identifies the target site in the acquired image, e.g., using pattern-matching techniques as described above with reference to Fig. 6. (Following the formation of a hole, the controller may identify the target site by using pattern-matching techniques to identify the hole.) Following the identification of the target site, the controller, at laser-aiming step 104, aims the laser at the target site. The controller then fires the laser at laser-firing step 106, such that treatment beams are emitted by the laser. The treatment beams may include, for example, a sequence of three or more pulses having a repetition rate of at least 20 Hz.

(Although not shown in Fig. 7, the controller typically uses an aiming beam to verify that the laser is aimed at the target site and/or to check the focus of the laser and/or of the camera, as described above with reference to Fig. 6.)

Following the firing of the laser, image-acquiring step 100 is again performed. Subsequently, the controller checks the acquired image for one or more properties deemed to indicate fluid flow through the target site. If the image possesses all the properties, the controller terminates the procedure. Otherwise, the controller returns to target-site-identifying step 102.

For example, as shown in Fig. 7, the controller may check, at a first checking step 108, whether the image shows a hole at the target site. If yes, the controller may compute the size of the hole and then check, at a second checking step 110, whether the hole is large enough, i.e., whether the size of the hole exceeds a predefined threshold. Subsequently, provided the hole is large enough, the controller may check, at a third checking step 112, whether the image shows debris near the target site, i.e., within a predefined distance from the target site. If yes, the controller may compute the size of the debris and then check, at a fourth checking step 114, whether the debris is large enough, i.e., whether the size of the debris exceeds a predefined threshold. If yes, the processor may check, at a fifth checking step 116, whether the images show movement of the debris. Subsequently, provided that movement of the debris is detected, the processor may terminate the procedure.

In some embodiments, following a failure of one of the checks, the controller may move the target site and/or modify the operating setting of the laser. For example, subsequently to ascertaining, at first checking step 108, that no hole was formed, the controller may increase the intensity of the laser. As another example, subsequently to ascertaining, at second checking step 110, that the hole is not large enough, the controller may, in performing target-site-identifying step 102, move the target site by a small distance such that the subsequent firing of the laser causes the hole to expand. Alternatively, the controller may first check whether the hole is expanding, by comparing the image to one or more previously-acquired images. In response to ascertaining that the hole is not expanding, the controller may move the target site; otherwise, the controller may maintain the location of the target site.

It will be appreciated by persons skilled in the art that the present invention is not limited to what has been particularly shown and described hereinabove. Rather, the scope of the present invention includes both combinations and subcombinations of the various features described hereinabove, as well as variations and modifications thereof that are not in the prior art, which would occur to persons skilled in the art upon reading the foregoing description.

## Claims

1. A system (20), comprising:
a laser (48), configured to irradiate a target site (41) in an iris (29) of an eye (25);
a camera (54), configured to acquire one or more images (23) of at least part of the iris;
**characterised in that** the system further comprises:
a controller (44), configured to:
identify, in the one or more images (23) of at least part of the iris, an indication of fluid flow through the target site, and
in response to identifying the indication, inhibit the laser from further irradiating the target site.

2. The system according to claim 1, wherein the target site is in a crypt in the iris.

3. The system according to claim 2, wherein the controller is further configured to, prior to the laser irradiating the target site:
identify the crypt in another image of at least part of the iris, and
in response to identifying the crypt, designate at least a portion of the crypt as the target site.

4. The system according to any one of claims 1-3, wherein the controller is configured to identify the indication by identifying debris within a predefined distance from the target site.

5. The system according to claim 4, wherein the controller is configured to identify the indication by identifying movement of the debris.

6. The system according to claim 4, wherein the controller is configured to identify the indication by identifying that a size of the debris is greater than a predefined threshold.

7. The system according to any one of claims 1-3, wherein the controller is configured to identify the indication by identifying a hole through the target site.

8. The system according to claim 7, wherein the controller is configured to identify the hole in response to a difference in color between the hole and a portion of the iris outside the hole.

9. The system according to claim 7, wherein the controller is configured to identify the indication by identifying that a size of the hole is greater than a predefined threshold.

10. The system according to claim 9, wherein the predefined threshold is a first predefined threshold, and wherein the controller is configured to identify the indication by identifying debris within a predefined distance from the target site and having a size greater than a second predefined threshold.

11. The system according to claim 10, wherein the second predefined threshold is a function of a size of the hole.

12. The system according to any one of claims 1-3, wherein the controller is further configured to:
identify the target site in other images of at least part of the iris, and
in response to identifying the target site, aim the laser at the target site.

13. A computer software product comprising a tangible non-transitory computer-readable medium in which program instructions are stored, which instructions, when run on a system as defined by claims 1-12 cause the controller to:
identify, in one or more images (23) of least part of an iris (29) of an eye (25), an indication of fluid flow through a target site (41) in the iris, and
in response to identifying the indication, inhibit a laser (48) from irradiating the target site.

14. The computer software product according to claim 13, wherein the instructions cause the controller to identify the indication by identifying debris within a predefined distance from the target site.

15. The computer software product according to claim 14, wherein the instructions cause the controller to identify the indication by identifying movement of the debris.

## Patentansprüche

1. System (20), das Folgendes umfasst:
einen Laser (48), der zum Bestrahlen einer Zielstelle (41) in einer Iris (29) eines Auges (25) konfiguriert ist;
eine Kamera (54), die zum Erfassen eines oder mehrerer Bilder (23) von wenigstens einem Teil der Iris konfiguriert ist;
**dadurch gekennzeichnet, dass** das System ferner Folgendes umfasst:
eine Steuerung (44), die zu Folgendem konfiguriert ist:
Identifizieren, in dem einen oder den mehreren Bildern (23) von wenigstens einem Teil der Iris, einer Indikation eines Fluidflusses durch die Zielstelle; und
als Reaktion auf das Identifizieren der Indikation, Hindern des Lasers daran, die Zielstelle weiter zu bestrahlen.

2. System nach Anspruch 1, wobei sich die Zielstelle in einer Krypte in der Iris befindet.

3. System nach Anspruch 2, wobei die Steuerung ferner zu Folgendem konfiguriert ist, bevor der Laser die Zielstelle bestrahlt:
Identifizieren der Krypte in einem anderen Bild wenigstens eines Teils der Iris, und
als Reaktion auf das Identifizieren der Krypte, Designieren wenigstens eines Teils der Krypte als die Zielstelle.

4. System nach einem der Ansprüche 1-3, wobei die Steuerung zum Identifizieren der Indikation durch Identifizieren von Trümmerstücken innerhalb einer vordefinierten Entfernung von der Zielstelle konfiguriert ist.

5. System nach Anspruch 4, wobei die Steuerung zum Identifizieren der Indikation durch Identifizieren einer Bewegung der Trümmerstücke konfiguriert ist.

6. System nach Anspruch 4, wobei die Steuerung zum Identifizieren der Indikation durch Identifizieren davon, dass eine Größe der Trümmerstücke größer als eine vordefinierte Schwelle ist, konfiguriert ist.

7. System nach einem der Ansprüche 1-3, wobei die Steuerung zum Identifizieren der Indikation durch Identifizieren eines Lochs durch die Zielstelle konfiguriert ist.

8. System nach Anspruch 7, wobei die Steuerung zum Identifizieren des Lochs als Reaktion auf eine Differenz einer Farbe zwischen dem Loch und einem Teil der Iris außerhalb des Lochs konfiguriert ist.

9. System nach Anspruch 7, wobei die Steuerung zum Identifizieren der Indikation durch Identifizieren davon, dass eine Größe des Lochs größer als eine vordefinierte Schwelle ist, konfiguriert ist.

10. System nach Anspruch 9, wobei die vordefinierte Schwelle eine erste vordefinierte Schwelle ist und wobei die Steuerung zum Identifizieren der Indikation durch Identifizieren von Trümmerstücken innerhalb einer vordefinierten Entfernung von der Zielstelle und mit einer Größe größer als eine zweite vordefinierte Schwelle konfiguriert ist.

11. System nach Anspruch 10, wobei die zweite vordefinierte Schwelle eine Funktion einer Größe des Lochs ist.

12. System nach einem der Ansprüche 1-3, wobei die Steuerung ferner zu Folgendem konfiguriert ist:
Identifizieren der Zielstelle in anderen Bildern von wenigstens einem Teil der Iris, und
als Reaktion auf das Identifizieren der Zielstelle, Zielen des Lasers auf die Zielstelle.

13. Computersoftwareprodukt, das ein greifbares nichtflüchtiges computerlesbares Medium umfasst, in dem Programmanweisungen gespeichert sind, wobei diese Anweisungen, wenn sie auf einem System nach einem der Ansprüche 1-12 ausgeführt werden, die Steuerung zu Folgendem veranlassen:
Identifizieren, in einem oder mehreren Bildern (23) von wenigstens einem Teil einer Iris (29) eines Auges (25), einer Indikation eines Fluidflusses durch eine Zielstelle (41) in der Iris, und
als Reaktion auf das Identifizieren der Indikation, Hindern eines Lasers (48) daran, die Zielstelle zu bestrahlen.

14. Computersoftwareprodukt nach Anspruch 13, wobei die Anweisungen die Steuerung zum Identifizieren der Indikation durch Identifizieren von Trümmerstücken innerhalb einer vordefinierten Entfernung von der Zielstelle veranlassen.

15. Computersoftwareprodukt nach Anspruch 14, wobei die Anweisungen die Steuerung zum Identifizieren der Indikation durch Identifizieren einer Bewegung der Trümmerstücke veranlassen.

## Revendications

1. Système (20), comprenant :
un laser (48), configuré pour exposer à un rayonnement un site cible (41) dans l'iris (29) d'un œil (25) ;
une caméra (54), configurée pour acquérir une ou plusieurs images (23) d'au moins une partie de l'iris ;
le système étant **caractérisé en ce qu'**il comprend en outre :
un contrôleur (44), configuré pour :
identifier, dans les une ou plusieurs images (23) d'au moins une partie de l'iris, une indication d'un écoulement fluidique à travers le site cible, et
en réponse à l'identification de l'indication, bloquer toute exposition supplémentaire à un rayonnement par le laser du site cible.

2. Système selon la revendication 1, le site cible se situant dans une crypte dans l'iris.

3. Système selon la revendication 2, le contrôleur étant configuré en outre pour, préalablement à l'exposition à un rayonnement par le laser du site cible :
identifier la crypte dans une autre image d'au moins une partie de l'iris, et
en réponse à l'identification de la crypte, désigner au moins une portion de la crypte comme site cible.

4. Système selon l'une quelconque des revendications 1 à 3, le contrôleur étant configuré pour identifier l'indication en identifiant des débris dans un rayon prédéfini du site cible.

5. Système selon la revendication 4, le contrôleur étant configuré pour identifier l'indication en identifiant un mouvement des débris.

6. Système selon la revendication 4, le contrôleur étant configuré pour identifier l'indication en identifiant qu'une taille des débris est supérieure à un seuil prédéfini.

7. Système selon l'une quelconque des revendications 1 à 3, le contrôleur étant configuré pour identifier l'indication en identifiant un trou à travers le site cible.

8. Système selon la revendication 7, le contrôleur étant configuré pour identifier le trou en réponse à une différence de couleur entre le trou et une portion de l'iris en dehors du trou.

9. Système selon la revendication 7, le contrôleur étant configuré pour identifier l'indication en identifiant qu'une taille du trou est supérieure à un seuil prédéfini.

10. Système selon la revendication 9, le seuil prédéfini étant un premier seuil prédéfini, et le contrôleur étant configuré pour identifier l'indication en identifiant des débris dans un rayon prédéfini du site cible et dont la taille est supérieure à un deuxième seuil prédéfini.

11. Système selon la revendication 10, le deuxième seuil prédéfini étant fonction d'une taille du trou.

12. Système selon l'une quelconque des revendications 1 à 3, le contrôleur étant configuré en outre pour :
identifier le site cible dans d'autres images d'au moins une partie de l'iris, et
en réponse à l'identification du site cible, pointer le laser sur le site cible.

13. Produit logiciel d'ordinateur comprenant un support tangible non transitoire lisible par ordinateur dans lequel sont stockées des instructions de programme, les instructions, lorsqu'elles s'exécutent sur une système selon l'une quelconque des revendications 1 à 12, amenant le contrôleur à :
identifier, dans une ou plusieurs images (23) d'au moins une partie de l'iris (29) d'un œil (25), une indication d'un écoulement fluidique à travers un site cible (41) dans l'iris, et
en réponse à l'identification de l'indication, bloquer toute exposition supplémentaire à un rayonnement par un laser (48) du site cible.

14. Produit logiciel d'ordinateur selon la revendication 13, les instructions amenant le contrôleur à identifier l'indication en identifiant des débris dans un rayon prédéfini du site cible.

15. Produit logiciel d'ordinateur selon la revendication 14, les instructions amenant le contrôleur à identifier l'indication en identifiant un mouvement des débris.
